# EUROPEAN PATENT SPECIFICATION

(11) **EP 1 660 121 B1**
(45) Date of publication and mention of the grant of the patent: **19.01.2011**
(21) Application number: 04786594.4
(22) Date of filing: 26.08.2004
(51) Int. Cl.: A61K 39/02, A61P 31/04, C12N 1/20

(54) **LIVE ATTENUATED BACTERIAL VACCINE**
ATTENUIERTE BAKTERIELLE LEBENDVAKZINE
VACCIN BACTERIEN ATTENUE VIVANT

(30) Priority: 29.08.2003 US 498988 P; 29.08.2003 US 498961 P
(43) Date of publication of application: 31.05.2006
(73) Proprietor: The Board of Governors for Higher Education, State of Rhode Island and Providence Plantations, Kingston, Rhode Island 02881 (US)
(72) Inventor: COHEN, Paul, S., Narragansett, RI 02882 (US)
(74) Representative: Janssen, Paulus J. P.
(86) International application number: PCT/US2004/027896
(87) International publication number: WO 2005/021031

(56) References cited:
- EP-A2- 1 074 266
- RITTER A ET AL: "The Pai-associated leuX specific tRNA5(Leu) affects type 1 fimbriation in pathogenic Escherichia coli by control of FimB recombinase expression." MOLECULAR MICROBIOLOGY. SEP 1997, vol. 25, no. 5, September 1997 (1997-09), pages 871-882, XP002316124 ISSN: 0950-382X
- NEWMAN J V ET AL: "Role of leuX in Escherichia coli colonization of the streptomycin-treated mouse large intestine." MICROBIAL PATHOGENESIS. NOV 1994, vol. 17, no. 5, November 1994 (1994-11), pages 301-311, XP002316125 ISSN: 0882-4010
- DATSENKO KIRILL A ET AL: "One-step inactivation of chromosomal genes in Escherichia coli K-12 using PCR products" PROCEEDINGS OF THE NATIONAL ACADEMY OF SCIENCES OF USA, NATIONAL ACADEMY OF SCIENCE. WASHINGTON, US, vol. 97, no. 12, 6 June 2000 (2000-06-06), pages 6640-6645, XP002210218 ISSN: 0027-8424

### SEQUENCE LISTING

<110> The Board of Governors for higher education, State of Rhode Island and Providence plantations
<120> Live attenuated bacterial vaccine
<130> LeuX
<160> 1
<170> PatentIn version 3.2
<210> 1
   <211> 210
   <212> DNA
   <213> Salmonella typhimurium
<400> 1
   acttagcgat acaggtgtat gctcgtgctt gcatggtggc gtatgacagg tataatccac 60
   aacgttttcc gcatacctct tcagtgccga agtggcgaaa tcggtagacg cagttgattc 120
   aaaatcaacc gtagaaatac gtgccggttc gagtccggcc ttcggcacca agtgatgcaa 180
   taattagctg cctaatggtg gctttttttg 210

## Description

The present invention relates to live attenuated bacteria for use in a medicament, to vaccines based upon such bacteria useful for the prevention of microbial pathogenesis, to the use of live attenuated bacteria for the manufacture of such vaccines and to methods for the preparation of such vaccines.

Immunity to microbial pathogenesis is one means by which a warm blooded animal avoids pathogenesis, or suffers a less intense pathogenic state. Incomplete immunity to a given pathogen results in morbidity and mortality in a population exposed to a pathogen. It is generally agreed that vaccines based on live but attenuated micro-organisms (live attenuated vaccines) induce a highly effective type of immune response. Such vaccines have the advantage that, once the animal host has been vaccinated, entry of the microbial pathogen into the host induces an accelerated recall of earlier, cell-mediated or humoral immunity which is able to control further growth of the organism before the infection can assume clinically significant proportions. Vaccines based on a killed pathogen (killed vaccine) are generally conceded to be unable to achieve this type of response. However, contrary to killed vaccines, vaccines that contain a live pathogen present, depending on the level of attenuation, the danger that the vaccinated host upon vaccination may contract the disease against which protection is being sought.

Vaccines against bacteria belonging to the closely related families of Escherichia and Salmonella follow the general rules given above. Many members of these families of bacteria are pathogenic due to the fact that they infect the digestive tract and the bladder. The pathogenic effect of these bacteria is closely related to their ability to colonise the mucosal layers of the digestive tract and the bladder. It is the phenomenon of colonisation that leads to the prolonged presence of the pathogen in the digestive tract and/or the bladder and to a very close contact of the pathogen to the mucosal layers, which can also lead to invasion of other tissues. Thus, at the same time, paradoxically, it's due to the fact that these bacteria colonise the digestive tract and the bladder, and thus at the same time cause disease, that the immune system is triggered to develop a certain level of immune response. This immune response is clearly developed too late to suppress the pathogenic effect of the colonising bacteria.

It would thus be desirable to have a vaccine that possesses the immunising attributes of a live micro-organism but that is not capable of causing undesirable side effects upon vaccination.

For live attenuated vaccines there is however the following paradox: an approach for attenuating bacteria is the removal of one or more virulence factors. In most cases however, virulence factors also play a role in inducing immunity. In those cases, deletion of virulence factors unavoidably impairs the immunogenic capacities of the bacterium. This is of course an unwanted situation. A live vaccine should preferably retain the antigenic complement of the wild-type strain, without being viulent.

It is an objective of the present invention to provide live attenuated vaccine that do not suffer from several of the drawbacks mentioned above.

Bacteria of the families Escherichia and Salmonella have several virulence factors. An example of a single gene involved in the synthesis of many virulence factors, including those playing a role in colonisation in both Escherichia and Salmonella, is the gene encoding LeuX. This gene encodes a specific tRNA: tRNA₅^{leu}. SEQ ID NO 1 provides the sequence of a *Salmonella typhimurium leu*X gene.

LeuX and its role in colonisation and fimbriae synthesis has been described i.a. by Newman et al., (FEMS Microbiology Letters 122: 281-287 (1994)) Newman et al.(Microbial Pathogenesis 17: 301-311 (1994)) and by Collighan, R.J. and Woodward M.J. (Vet. Microbiol. 80: 235-245 (2001))

Ritter et al., in Mol. Microbiol 17: 109-121 (1995) have thoroughly analysed the various roles of the LeuX gene product in virulence. They showed that the presence of LeuX is crucial for the stimulation of the synthesis of Type 1 fimbriae and flagella, both involved in motility and colonisation, and is crucial for the synthesis of proteins involved in iron uptake, for the synthesis of enterobactin and for *in vitro* virulence (see also the european patent application EP 1 074 266 and Ritter et al., in Mol. Microbiol 25: 871-882, 1997).

All these characteristics are known to strongly contribute to virulence. Therefore, they are at the same time the most important targets for an immune response. An immune response against fimbriae and flagella would interfere with colonisation and motility, whereas an immune response against enterobactin and proteins involved in iron uptake would block toxic effects and deprive the bacterium from the possibility to obtain essential iron respectively.

Thus, from a vaccine point of view, the key virulence factors of choice to be present in a vaccine would be the virulence factors expressed in the presence of LeuX. The vaccine of choice would therefore (preferably) be a subunit vaccine comprising type 1 fimbriae, flagella, enterobactin and proteins involved in iron uptake. Such a vaccine would first of all be safe, and secondly most likely induce immunity against these 4 virulence factors and by doing so provide protection against infection.

Live attenuated strains from which the LeuX gene is deleted, do not make the virulence factors mentioned above. If such LeuX-negative deletion-mutants were to be used in a vaccine, they would not induce protection against these most crucial virulence factors: type 1 fimbriae, flagella, enterobactin and proteins involved in iron uptake.

Given this fact, LeuX is to be considered as a highly unattractive candidate for deletion in a live attenuated vaccine strain.

Moreover, bacteria that lack colonising abilities (due to lack of type 1 fimbriae and flagella) as would be the case for LeuX-negative deletion-mutants, will not be expected to come into close contact with the host cells, and will consequently be assumed to be washed out quickly. Therefore, they would even not be expected to induce any substantial immunity against those virulence factors that would still be present in the absence of the LeuX gene product.

Surprisingly, however, it was found now that bacterial strains, both from the family of Escherichia and Salmonella, not having a functional tRNA₅^{leu} are very well capable of inducing a protective immune response against virulent wild-type bacteria in the host animal. This is indeed against all expectations, since they do not induce any immunity against the key virulence factors mentioned above.

Therefore, a first embodiment of the present invention relates to live attenuated bacteria that have no functional tRNA₅^{leu}, for use in a vaccine.

Dobrindt U., et al., (FEMS Microbiology letters 162: 135-141 (1998)) have confirmed that it is indeed the LeuX-encoded *tRNA₅^{leu}* that is responsible for e.g. survival of a uropathogenic E. coli in mouse bladder mucus, and not the presence of the pathogenicity islands as such. These pathogenicity islands are the regions that encode e.g. haemolysins, fimbrial adhesins and so on.

Due to its key position in the bacterial pathogenicity, the *tRNA₅^{leu}* gene and its gene product tRNA₅^{leu} are widespread in the bacterial realm. The *tRNA₅^{leu}* is highly conserved. It can be found in e.g. *Escherichia coli* and in *Salmonella enterica* species, such as serotype *typhimurium, enteritidis, galinarum* and *dublin* and in *Yersinia* species such as *Y. pestis.*

The gene itself and its complete nucleotide sequence in *Salmonella* and *Escherichia* are discussed i.a. by Thorbjarnardottir, S. et al. (J. Bacteriology 161: 219-222 (1985)), Yoshimura, M. et al. (J. Mol. Biol. 177: 627-644 (1984)), Andersson, S. et al. (microbial Review 54: 198-210 (1990)), Komine, Y. et al., (J. Mol. Biol. 212: 579-598 (1990)) and Blum, G. et al. (Infect. & Immun. 62: 606-614 (1994)).

A functional *tRNA₅^{leu}* is understood to be a *tRNA₅^{leu}* having the characteristics of the wild-type *tRNA₅^{leu}*, i.e.: is capable of adding the amino acid Leucine to a protein strand during its synthesis, if the codon encoding leucine is UUG. Therefore, a *tRNA₅^{leu}* that is defective in at least this function is considered to be a non-functional *tRNA₅^{leu}*.

Live attenuated bacteria according to the invention can also be obtained by introducing a mutation in the LeuX gene that prevents the synthesis of functional tRNA₅^{leu}.

Therefore, a preferred embodiment of the present invention relates to live attenuated bacteria not having a functional tRNA₅^{leu} as a result of a mutation in the *leux* gene, for use in a vaccine.

Such a mutation can be an insertion, a deletion, a substitution or a combination thereof, provided that the mutation leads to the failure to express a functional *tRNA₅^{leu}.*

Live attenuated bacteria for use according to the invention can be obtained in several ways. One possible way of obtaining such bacteria is by means of classical methods such as the treatment of wild-type bacteria having the *tRNA₅^{leu}* gene with mutagenic agents such as base analogues, treatment with ultraviolet light or temperature treatment.

Strains according to the invention can be easily selected on the basis that they would lack at least the four virulence factors mentioned above.

The nature of the mutation caused by classical mutation techniques would however be unknown. This may be a point mutation in the LeuX gene which may, although this is unlikely to happen, eventually revert to wild-type.

In order to avoid this small risk, transposon mutagenesis would be a good alternative. Mutagenesis by transposon mutagenesis, is also a mutagenesis-technique well-known in the art. This is a mutation accomplished at a localised site in the chromosome. Transposon-insertions can not be targeted to a specific gene. It is however easy to pick up the LeuX mutants since they would lack at least the four virulence factors mentioned above.

A much more elegant possibility to introduce a mutation, now at a predetermined site, rather deliberately than randomly, is offered by recombinant DNA-technology. Such a mutation may again be an insertion, a deletion, a replacement of one nucleotide by another one or a combination thereof, with the only proviso that the mutated gene no longer encodes functional *tRNA₅^{leu}.* Such a mutation can e.g. be made by deletion of a number of base pairs. Even very small deletions such a stretches of 10 base pairs can already render *tRNA₅^{leu}* non-functional. Even the deletion of one single base pair may already lead to a non-functional *tRNA₅^{leu}.* More preferably, a longer stretch is removed, e.g. 50 or more base pairs. Even more preferably, the whole *tRNA₅^{leu}* gene is deleted.

All techniques for the construction of *tRNA₅^{leu}* -negative mutants are well-known standard techniques. They relate to cloning of the tRNA₅^{leu} gene, modification of the gene sequence by site-directed mutagenesis, restriction enzyme digestion followed by re-ligation or PCR-approaches and to subsequent replacement of the wild type *tRNA₅^{leu}* gene with the mutant gene (allelic exchange or allelic replacement). Standard recombinant DNA techniques such as cloning the *tRNA₅^{leu}* gene in a plasmid, digestion of the gene with a restriction enzyme, followed by endonuclease treatment, re-ligation and homologous recombination in the host strain, are all known in the art and described i.a. in Maniatis/Sambrook (Sambrook, J. et a/. Molecular cloning: a laboratory manual. ISBN 0-87969-309-6). Site-directed mutations can e.g. be made by means of in vitro site directed mutagenesis using the Transformed kit sold by Clontech. PCR-techniques are extensively described in (Dieffenbach & Dreksler; PCR primers, a laboratory manual. ISBN 0-87969-447-3 and ISBN 0-87969-447-5).

The *tRNA₅^{leu}* gene comprises not only the coding sequence encoding *tRNA₅^{leu},* but also regulatory sequences such as the promoter. Therefore, not only mutations in the coding regions but also mutations in those sequences essential for correct transcription are considered to fall within the scope of the invention.

In a more preferred embodiment, the invention relates to live attenuated bacteria of the genera *Escherichia* and *Salmonella.*

In an even more preferred form of the invention, the live attenuated bacterium according to the invention is selected from the group consisting of *S. enterica* serotype *typhimurium, enteritidis, choleraesuis, dublin, typhi, gallinarum, abortusovi, abortus-equi, pullorum, E. coli* or *Y. pestis.* These bacterial genera comprise a large number of species that are pathogenic to both humans and a variety of different animals.

In a still even more preferred form thereof, the live attenuated bacterium according to the invention is *S*. *enterica, E. coli* or *Y. pestis.*

In an even further preferred form, this embodiment relates to live attenuated bacteria according to the invention in which the mutation in the *tRNA₅^{leu}* gene has been made by recombinant DNA technology.

Well-defined and deliberately made mutations involving the deletion of fragments of the *tRNA₅^{leu}* gene or even the whole gene or the insertion of heterologous DNA-fragments or both, have the advantage, in comparison to classically induced mutations, that they will not revert to the wild-type situation.

Thus, in a most preferred form, this embodiment of the invention refers to live attenuated bacteria in which the *tRNA₅^{leu}* gene comprises an insertion and/or a deletion.

Given the large amount of vaccines given nowadays to both pets and farm animals, it is clear that combined administration of several vaccines would be desirable, if only for reasons of decreased vaccination costs. It is therefore very attractive to use live attenuated bacteria as a recombinant carrier for heterologous genes, encoding antigens selected from other pathogenic micro-organisms or viruses. Administration of such a recombinant carrier has the advantage that immunity is induced against two or more diseases at the same time. The live attenuated bacteria for use in a vaccine, according to the present invention provide very suitable carriers for heterologous genes, since the gene encoding *tRNA₅^{leu}* can be used as an insertion site for such heterologous genes. The use of the *tRNA₅^{leu}* gene as an insertion site has the advantage that at the same time the *tRNA₅^{leu}* gene is inactivated and the newly introduced heterologous gene can be expressed (in concert with the homologous bacterial genes). The construction of such recombinant carriers can be done routinely, using standard molecular biology techniques such as allelic exchange. Therefore, another embodiment of the invention relates to live attenuated recombinant carrier bacteria, preferably of the genera *Escherichia, Salmonella* and *Yersinia* that do not produce a functional *tRNA₅^{leu},* and in which a heterologous gene is inserted.

Such a heterologous gene can, as mentioned above, e.g. be a gene encoding an antigen selected from other pathogenic micro-organisms or viruses. Such genes can e.g. be derived from pathogenic herpesviruses (e.g. the genes encoding the structural proteins of herpesviruses), retroviruses (e.g. the gp160 envelope protein), adenoviruses and the like. Also a heterologous gene can be obtained from pathogenic bacteria. As an example, genes encoding protective antigens such as bacterial toxins like Actinobacillus pleuropneumoniae toxins, Clostridium toxins, outer membrane proteins and the like are very suitable bacterial heterologous genes.

Another possibility is to insert a gene encoding a protein involved in triggering the immune system, such as a cytokine, an interleukin or an interferon, or another gene involved in immune-regulation.

Insertion of the heterologous gene in the *tRNA₅^{leu}* gene is advantageous, since in that case there is no need to find a new suitable insertion site for the heterologous gene, and at the same time the *tRNA₅^{leu}* gene is knocked out.

Thus, in a preferred form of this embodiment the heterologous gene is inserted in the *tRNA₅^{leu}* gene. The heterologous gene can be inserted somewhere in the *tRNA₅^{leu}* gene or it can be inserted at the site of the *tRNA₅^{leu}* gene while this gene has been partially or completely deleted.

Because of their unexpected attenuated but immunogenic character *in vivo,* the bacteria for use in a vaccine, according to the invention are very suitable as a basis for live attenuated vaccines. Thus, still another embodiment of the invention relates to such live attenuated vaccines for the protection of animals and humans against *Escherichia, Yersinia* or *Salmonella* infection or the pathogenic effects thereof, that comprise a bacterium of which the wild type form comprises a *tRNA₅^{leu}* gene.

Such vaccines comprise an immunogenically effective amount of a live attenuated bacterium according to the invention or a live recombinant carrier bacterium according to the invention, and a pharmaceutically acceptable carrier.

Preferably, the vaccine comprises a live attenuated bacterium according to the invention, selected from the group of *Escherichia, Salmonella* and *Yersinia.*

Immunogenically effective means that the amount of live attenuated bacteria administered at vaccination is sufficient to induce in the host an effective immune response against virulent forms of the bacterium.

In addition to an immunogenically effective amount of the live attenuated bacterium described above, a vaccine according to the present invention also contains a pharmaceutically acceptable carrier. Such a carrier may be as simple as water, but it may e.g. also comprise culture fluid in which the bacteria were cultured. Another suitable carrier is e.g. a solution of physiological salt concentration.

The useful dosage to be administered will vary depending on the age, weight and animal vaccinated, the mode of administration and the type of pathogen against which vaccination is sought.

The vaccine may comprise any dose of bacteria, sufficient to evoke an immune response. Doses ranging between 10³ and 10¹⁰ bacteria are e.g. very suitable doses.

Optionally, one or more compounds having adjuvant activity may be added to the vaccine. Adjuvants are non-specific stimulators of the immune system. They enhance the immune response of the host to the vaccine. Examples of adjuvants known in the art are Freunds Complete and Incomplete adjuvant, vitamin E, non-ionic block polymers, muramyldipeptides, ISCOMs (immune stimulating complexes, cf. for instance European Patent EP 109942), Saponins, mineral oil, vegetable oil, and Carbopol.

Adjuvants, specially suitable for mucosal application are e.g. the *E*. *coli* heat-labile toxin (LT) or *Cholera* toxin (CT).

Other suitable adjuvants are for example aluminium hydroxide, aluminium phosphate or aluminium oxide, oil-emulsions (e.g. of Bayol F ^{(R)} or Marcol 52 ^{(R)}), saponins or vitamin-E solubilisate.

Therefore, in a preferred form, the vaccines according to the present invention comprise an adjuvant.

Other examples of pharmaceutically acceptable carriers or diluents useful in the present invention include stabilisers such as SPGA, carbohydrates (e.g. sorbitol, mannitol, starch, sucrose, glucose, dextran), proteins such as albumin or casein, protein containing agents such as bovine serum or skimmed milk and buffers (e.g. phosphate buffer).

Especially when such stabilisers are added to the vaccine, the vaccine is very suitable for freeze-drying. Therefore, in a more preferred form, the vaccine is in a freeze-dried form.

Still another embodiment relates to the use of a bacterium according to the invention for the manufacture of a vaccine for the protection of animals and humans against infection with a wild type bacterium or the pathogenic effects of infection.

For administration to animals or humans, the vaccine according to the present invention can be given inter alia intranasally, intradermally, subcutaneously, orally, by aerosol or intramuscularly. For application to poultry, wing web and eye-drop administration are very suitable.

The skilled person would know how to administer a vaccine according to the invention, because the method would most likely not differ from the methods followed for vaccination with presently existing bacterial vaccines. A vaccine according to the invention, especially when it comprises bacteria belonging to the family of *E*. *coli, Salmonella* or *Yersinia* would preferably be given orally.

Still another embodiment of the invention relates to methods for the preparation of a vaccine according to the invention. Such methods comprise the admixing of a live attenuated bacterium according to the invention or a live recombinant carrier bacterium according to the invention, and a pharmaceutically acceptable carrier.

### EXAMPLES

### Example 1

### Construction of leuX mutants

The Wanner system (Proc. Natl. Acad. Sci. U.S.A. June 6, 2000. 97(12):6640-45.) was used to replace the *S*. *typhimurium* SR-1*1 leu*X gene with a kanamycin resistance gene.

A wild-type *S. typhimurium;* SR-11 was used in this example, but the principle described is equally applicable to all bacteria carrying the *leuX* gene.

Sequences homologous to *S. typhimurium* SR-11 *leuX* flanking sequences were added to the 5' end of primers to a kanamycin cassette. Forward Primer: 5' GGT ATA ATC CAC AAC GTT TTCCGC ATA CCT CTT CAG gtgtaggctggagctgcttcg and Reverse Primer: 5' AAA AAG CCA CCA TTA GGC AGC TAA TTA TTG CAT CAC catatgaatatcctccttag (upper case letters are *leuX* sequences and lower case letters are the kanamycin primers) were used to PCR the kanamycin cassette from the plasmid pKD4. To amplify the kanamycin (∼1700bp) cassette from the plasmid pKD4, the primer set containing the priming sites were used in a standard PCR reaction with Fisher Taq DNA polymerase (1.5mM MgCl₂). Cycling conditions were 1X 94°C 4min; 35X 94°C 30sec, 55°C 15sec, 72°C 75-105sec; 1X 72°C 7min. Eight 100 µl reactions were pooled, 5µl was checked on a gel, and the linear PCR product was ethanol precipitated and resuspended in 2-4 µl of water.

*S. typhimurium* SR-11 cells were electroporated with the temperature sensitive plasmid pKD46. When these cells are grown at 30°C in the presence of arabinose the plasmid expresses the lambda Red recombinase. The cells (A₆₀₀=0.6) were made competent for electroporation by centrifugation and washing 3-4X with cold 10% glycerol. The linear PCR product was then electroporated into the competent cells. The recombinase acts to replace the wild type *leuX* gene with the deleted *leuX* gene containing the kanamycin cassette. *S. typhimurium* SR-11 clones containing the deleted *leuX* gene were selected by overnight growth at 37°C on Luria agar plates containing kanamycin (40 µg/ml), which also resulted in loss of the temperature sensitive pKD46 plasmid.

Primers 5' and 3' of the *leuX* deletion/antibiotic cassette insertion, i.e. upstream and downstream, were used to verify the mutants. For the *S. typhimurium* SR-11 *leuX* mutant, primers containing a *Pstl* site and homologous to regions 5' and 3' of the *leuX* gene (the primers are called Pst Leux 5': ctagctgcag gcgttaatctgctggagaaggc; and Pst Leux 3': ctagctgcag acgaccaacacggaaagaccac) were used to amplify the expected approximate 1700bp band (versus a 400bp wildtype band). 2.5mM MgCl₂, with Finnzyme DyNAzymell polymerase was used in the reaction. Cycling conditions were 1X 94°C 4min; 30X 94°C 30sec, 55°C 30sec, 72°C 105sec; 1X 72°C 7min.

### Example 2

### Animal tests

### Experimental design

To test both safety and efficacy, broilers were inoculated by spray on day of hatch and inoculated orally at 15 days of age with approximately 10⁷ CFU of SR11-LeuX⁽⁻⁾ (The LeuX-minus mutant).

Safety was assessed by clinical observation after vaccination. Also, cloacal swabs were taken at days 8, 15, 22 and 30 to determine the presence of the vaccine strain in the intestinal tract. Swabs were used to inoculate Brilliant Green Agars (BGA) directly and after enrichment in Rappaport Vassiliades Broth. At 30 days of age, 5 vaccinated animals were necropsied and their livers and spleens were cultured to determine if the vaccine strain had invaded from the intestinal track.

To test for efficacy, the animals received an oral challenge infection with 1.4x10⁶ CFU of a tetracycline resistant wild-type S.t. strain at 30 days of age. Two weeks after challenge infection, the animals were euthanized and the livers, spleens, cloacal swabs and swabs of the cecum contents were cultured for the challenge strain. Organs and swabs were inoculated on BGA containing tetracycline (BGAtet) directly and also after incubation in an enrichment medium (buffered peptone water containing tetracycline).

### Animals

Hatching eggs were obtained from a Salmonella free broiler breeder flock.

### Results

No clinical abnormalities were observed after the spray and oral vaccinations. The SR11-LeuX⁽⁻⁾ strain was cultured from cloacal swabs of some of the vaccinated animals on days 8, 15, 22 and 30. At 30 days of age, the vaccine strain was still shed by 53% of the vaccinated animals, but it was not reisolated from the livers and spleens of the 5 animals that were necropsied.

As shown in Table 1, the challenge strain was isolated from the liver and spleen of non-vaccinated animals. In addition, the challenge strain was reisolated from the cloaca and caecum of practically all non-vaccinated control animals.

Vaccination with SR11-LeuX⁽⁻⁾ resulted in complete clearance of the challenge strain 14 days post infection. In addition, no challenge strain was found in the liver and spleen of vaccinated animals.

The results show, that Lieux⁽⁻⁾ bacteria, even if the LeuX⁽⁻⁾ mutation is the only attenuating mutation, are safe and efficacious in live vaccines.

**Table 1**

| | S.t. (tet^{r}) positive | |
|---|---|---|
| Group | SR11-LeuX⁽⁻⁾ | Control |
| Spleen | 0/14^{a} | 10/12 |
| Liver | 0/14^{a} | 10/12 |
| Cloaca | 0/14^{a} | 8/12 |
| Caecum | 0/14^{a} | 11/12 |

| | | |
|---|---|---|
| ^{a}: significantly different from control (p<0.05, Fisher exact test) | | |

## Claims

1. Live attenuated bacterium of the genus *Escherichia, Yersinia* or *Salmonella,* said bacterium not having a functional tRNA₅^{leu}, for use in a vaccine.

2. Live attenuated bacterium for use according to claim 1, said bacterium not having a functional tRNA₅^{leu} as a result of a mutation in the *leux* gene.

3. Live attenuated bacterium for use according to claim 1 or 2, wherein said bacterium is selected from the group consisting of *E*. *coli, S. enterica* serotype *typhimurium, enteritidis, choleraesuis, dublin, typhi, gallinarum, abortusovi, abortus-equi* or *pullorum.*

4. Live attenuated bacterium for use according to claim 1-3, **characterised in that** the mutation comprises an insertion and/or a deletion.

5. Live attenuated bacterium for use according to clams 1-4, **characterised in that** said bacterium carries a heterologous gene.

6. Live attenuated bacterium for use according to claim 5 **characterised in that** the heterologous gene is inserted in the *leux* gene.

7. Live attenuated vaccine for the protection of animals and humans against infection with a pathogenic bacterium or the pathogenic effects thereof, **characterised in that** said vaccine comprises a bacterium as defined in claims 1-6 and a pharmaceutically acceptable carrier.

8. Live attenuated vaccine according to claim 7, **characterised in that** it comprises an adjuvant.

9. Live attenuated vaccine according to claim 7 or 8, **characterised in that** it is in a freeze-dried form.

10. Use of a live attenuated bacterium as defined in claims 1-6 for the manufacture of a vaccine for the protection of animals against infection with a pathogenic bacterium or the pathogenic effects of infection.

11. Method for the preparation of a vaccine according to claims 7-9, **characterised in that** said method comprises the admixing of a live attenuated bacterium as defined in claims 1-6 and a pharmaceutically acceptable carrier.

## Patentansprüche

1. Attenuiertes Lebendbakterium des Genus *Escherichia, Yersinia* oder *Salmonella,* wobei das Bakterium keine funktionelle tRNA₅^{leu} aufweist, zur Verwendung in einer Vakzine.

2. Attenuiertes Lebendbakterium zur Verwendung nach Anspruch 1, wobei das Bakterium aufgrund einer Mutation im *leux*-Gen keine funktionelle tRNA₅^{leu} aufweist.

3. Attenuiertes Lebendbakterium zur Verwendung nach Anspruch 1 oder 2, wobei das Bakterium ausgewählt ist aus der Gruppe *E. coli, S. enterica-Serotyp typhimurium, enteritidis, choleraesuis, dublin, typhi, gallinarum, abortusovi, abortus-equi* oder *pullorum.*

4. Attenuiertes Lebendbakterium zur Verwendung nach Anspruch 1-3, **dadurch gekennzeichnet, dass** die Mutation eine Insertion und/oder eine Deletion umfasst.

5. Attenuiertes Lebendbakterium zur Verwendung nach Anspruch 1-4, **dadurch gekennzeichnet, dass** das Bakterium ein heterologes Gen trägt.

6. Attenuiertes Lebendbakterium zur Verwendung nach Anspruch 5, **dadurch gekennzeichnet, dass** das heterologe Gen im leux-Gen inseriert ist.

7. Attenuierte Lebendvakzine zum Schutz von Menschen und Tieren gegen Infektion mit einem krankheitserregenden Bakterium oder die krankheitserregenden Effekte davon, **dadurch gekennzeichnet, dass** die Vakzine ein Bakterium mit der in Anspruch 1-6 angegebenen Bedeutung sowie einen pharmazeutisch unbedenklichen Trägerstoff umfasst.

8. Attenuierte Lebendvakzine nach Anspruch 7, **dadurch gekennzeichnet, dass** sie ein Adjuvans umfasst.

9. Attenuierte Lebendvakzine nach Anspruch 7 oder 8, **dadurch gekennzeichnet, dass** sie in gefriergetrockneter Form vorliegt.

10. Verwendung eines attenuierten Lebendbakteriums mit der in Anspruch 1-6 angegebenen Bedeutung zur Herstellung einer Vakzine zum Schutz von Tieren gegen Infektion mit einem krankheitserregenden Bakterium oder die krankheitserregenden Effekte davon.

11. Verfahren zur Herstellung einer Vakzine nach Anspruch 7-9, **dadurch gekennzeichnet, dass** man bei dem Verfahren ein attenuiertes Lebendbakterium mit der in Anspruch 1-6 angegebenen Bedeutung sowie einen pharmazeutisch unbedenklichen Trägerstoff vermengt.

## Revendications

1. Bactérie atténuée vivante du genre *Escherichia, Yersinia* ou *Salmonella,* ladite bactérie n'ayant pas d'ARNt₅^{leu} fonctionnel, pour utilisation dans un vaccin.

2. Bactérie atténuée vivante pour utilisation selon la revendication 1, ladite bactérie n'ayant pas d'tRNA₅^{leu} fonctionnel en conséquence d'une mutation dans le gène *leux.*

3. Bactérie atténuée vivante pour utilisation selon la revendication 1 ou 2, ladite bactérie étant choisie dans le groupe consistant en *E. coli, S. enterica* sérotype *typhimurium, enteritidis, choleraesuis, dublin, typhi, gallinarum, abortus ovi, abortus-equi* ou *pullorum.*

4. Bactérie atténuée vivante pour utilisation selon les revendications 1-3, **caractérisée en ce que** la mutation comprend une insertion et/ou une délétion.

5. Bactérie atténuée vivante selon les revendications 1-4, **caractérisée en ce que** ladite bactérie porte un gène hétérologue.

6. Bactérie atténuée vivante pour utilisation selon la revendication 5, **caractérisée en ce que** le gène hétérologue est inséré dans le gène *leux.*

7. Vaccin atténué vivant pour la protection d'animaux et d'humains vis-à-vis d'une infection par une bactérie pathogène ou des effets pathogènes de cette dernière, **caractérisé en ce que** ledit vaccin comprend une bactérie telle que définie dans les revendications 1-6 et un support pharmaceutiquement acceptable.

8. Vaccin atténué vivant selon la revendication 7, **caractérisé en ce qu'**il comprend un adjuvant.

9. Vaccin atténué vivant selon la revendication 7 ou 8, **caractérisé en ce qu'**il se présente sous forme lyophilisée.

10. Utilisation d'une bactérie atténuée vivante telle que définie dans les revendications 1-6 pour la fabrication d'un vaccin pour la protection d'animaux à l'encontre d'une infection par une bactérie pathogène, ou des effets pathogènes d'une infection.

11. Procédé de préparation d'un vaccin selon les revendications 7-9, **caractérisé en ce que** ledit procédé comprend le mélange d'une bactérie atténuée vivante selon les revendications 1-6 et d'un support pharmaceutiquement acceptable.
